# EUROPÄISCHE PATENTSCHRIFT

(11) **EP 4 291 620 B1**
(45) Veröffentlichungstag und Bekanntmachung des Hinweises auf die Patenterteilung: **26.06.2024**
(21) Anmeldenummer: 22709285.5
(22) Anmeldetag: 15.02.2022
(51) Int. Cl.: C10G 9/36, C10G 9/00, F25J 3/02

(54) **VERFAHREN UND ANLAGE ZUR HERSTELLUNG VON ETHYLEN UND/ODER ANDEREN OLEFINEN DURCH STEAMCRACKEN**
METHOD AND SYSTEM FOR PRODUCING ETHYLENE AND / OR OTHER OLEFINS BY STEAM CRACKING
PROCÉDÉ ET INSTALLATION DE PRODUCTION D'ÉTHYLÈNE ET/OU D'AUTRES OLÉFINES PAR VAPOCRAQUAGE

(30) Priorität: 15.02.2021 EP 21157138
(43) Veröffentlichungstag der Anmeldung: 20.12.2023
(73) Patentinhaber: Linde GmbH, 82049 Pullach (DE); BASF SE, 67056 Ludwigshafen am Rhein (DE)
(72) Erfinder: KELLER, Benedikt, 80339 München (DE); BREHM, Peter, 85716 Unterschleißheim (DE); PRUNNER, Clemens, 85646 Neufarn (DE); REYNEKE, Hendrik, 81479 München (DE); KAMANN, Martin, 82041 Oberhaching (DE); GARBE, Gunter, 67688 Rodenbach (DE); KECK, Daniel, 68775 Ketsch (DE); SCHIETEKAT, Carl, 9100 Sint-Niklaas (BE); SPERBER, Axel, 67069 Ludwigshafen (DE); WECK, Alexander, 67126 Hochdorf-Assenheim (DE)
(86) Internationale Anmeldenummer: PCT/EP2022/053626
(87) Internationale Veröffentlichungsnummer: WO 2022/171894

(56) Entgegenhaltungen:
- EP-A1- 3 730 592

## Beschreibung

Die Erfindung betrifft ein Verfahren und eine Anlage zur Herstellung von Ethylen und/oder von anderen Olefinen durch Steamcracken gemäß den Oberbegriffen der jeweiligen unabhängigen Patentansprüche.

### Hintergrund der Erfindung

Verfahren und Anlagen zum Steamcracken von Kohlenwasserstoffen sind beispielsweise im Artikel "Ethylene" in Ullmann's Encyclopedia of Industrial Chemistry, Onlineausgabe, 15. April 2009, DOI: 10.1002/14356007.a10_045.pub2, beschrieben. Das Steamcracken (engl. Steam Cracking, im Deutschen auch als Dampfspalten bezeichnet) wird vorwiegend zur Gewinnung von kurzkettigen Olefinen wie Ethylen und Propylen, Diolefinen wie Butadien oder von Aromaten eingesetzt, ist jedoch nicht auf die Gewinnung solcher Verbindungen beschränkt.

Beim Steamcracken werden Komponentengemische (auch als Spaltgase oder Rohgase bezeichnet) erhalten, die zur Gewinnung der gewünschten Einzelkomponenten geeigneten Aufbereitungssequenzen unterworfen werden. Typischerweise erfolgt dabei in einem ersten Abschnitt (engl. Front-End Section) einer entsprechenden Aufbereitungssequenz eine Entfernung schwerer Verbindungen, falls vorhanden, und danach eine sogenannte Rohgasverdichtung, Sauergasentfernung und Trocknung. Nach der Aufbereitung in dem ersten Abschnitt erfolgt eine Fraktionierung, in der durch thermische Trennverfahren unter Einsatz von Ethylen- bzw. C2-Kältemittel und Propylen- bzw. C4-Kältemittel Fraktionen gebildet und bei Bedarf weiter aufgetrennt werden. Zu Details sei auf den erwähnten Artikel "Ethylene" in Ullmann's Encyclopedia of Industrial Chemistry, insbesondere die Abschnitte 5.3.2.1, "Front-End Section", und 5.3.2.2, "Hydrocarbon Fractionation Section", verwiesen.

In einer Ausgestaltung einer entsprechenden Fraktionierung, die auch im Zusammenhang mit der vorliegenden Erfindung zum Einsatz kommen kann, erfolgt in der Fraktionierung zunächst eine Trennung von Kohlenwasserstoffen mit zwei Kohlenstoffatomen und tiefer siedenden Komponenten wie Methan und Wasserstoff von Kohlenwasserstoffen mit drei Kohlenstoffatomen und höher siedenden Verbindungen. Ein derartiger Schritt wird üblicherweise auch als Deethanisierung bezeichnet, die Ausgestaltung einer entsprechenden Fraktionierung als "Deethanizer-First"- oder "Frontend-Deethanizer"-Verfahren.

Die in der Deethanisierung gasförmig erhaltene Fraktion von Kohlenwasserstoffen mit zwei Kohlenstoffatomen und tiefer siedenden Komponenten kann einer weiteren Trennung zugeführt werden, in der die Kohlenwasserstoffe mit zwei Kohlenstoffatomen von den weiter enthaltenen tiefer siedenden Komponenten abgetrennt werden. Ein derartiger Schritt wird auch als Demethanisierung bezeichnet. In einem "Deethanizer-First"- oder "Frontend-Deethanizer"-Verfahren ist also die Demethanisierung der Deethanisierung nachgeschaltet.

In alternativen Verfahren können die Schritte der Deethanisierung und der Demethanisierung auch in umgekehrter Reihenfolge durchgeführt werden. Man spricht dann von "Demethanizer-First"- oder "Frontend-Demethanizer"-Verfahren. Weitere Verfahrensvarianten sind in der erwähnten Fachliteratur beschrieben.

Im Zuge entsprechender Aufarbeitungssequenzen kommen an unterschiedlichen Stellen Verdichter zum Einsatz. Insbesondere wird dabei die Rohgasverdichtung unter Verwendung eines Rohgasverdichters (engl. Cracked Gas Compressor, CGC) vorgenommen und bei der Bereitstellung der Ethylen- bzw. C2-Kältemittel und Propylen- bzw. C3-Kältemittel kommen ein sogenannter Ethylenkältemittelverdichter (engl. Ethylene Refrigerant Compressor, ERC) und ein sogenannter Propylenkältemittelverdichter (engl. Propylene Refrigerant Compressor, ERC) zum Einsatz. Nachfolgend werden diese Begriffe verwendet, wenngleich in einem Ethylenkältemittelverdichter auch ggf. Ethan und in einem Propylenkältemittelverdichter auch ggf. Propan verdichtet werden kann. Produktfraktionen der thermischen Trennung können einer Verdichtung unter Verwendung weiterer Verdichter, sogenannter Produktverdichter, unterworfen werden.

In der EP 3 730 592 A1 ist eine Olefin-Syntheseanlage beschrieben. Diese umfasst einen Einsatzvorbehandlungsabschnitt, der so konfiguriert ist, dass er einen Einsatzstrom vorbehandelt, und einen Pyrolyseabschnitt, der einen oder mehrere Pyrolysereaktoren umfasst, die so konfiguriert sind, dass sie Kohlenwasserstoffe in dem Beschickungsstrom in Gegenwart eines Verdünnungsmittels spalten, um einen Spaltgasstrom zu erzeugen. Ein primärer Fraktionierungs- und Verdichtungsabschnitt ist vorgesehen, der so konfiguriert ist, dass er eine Wärmerückgewinnung aus dem Spaltgasstrom und ein Quenchen des Spaltgasstroms bereitstellt, eine Komponente aus dem Spaltgasstrom entfernt, und den Spaltgasstrom verdichtet, wodurch ein verdichteter Spaltgasstrom bereitgestellt wird. Alternativ oder zusätzlich kann ein Produkttrennungsabschnitt vorgesehen sein, der so konfiguriert ist, dass er einen Produktolefinstrom von dem komprimierten Spaltgasstrom trennt. Die Olefin-Syntheseanlage ist so konfiguriert, dass im Vergleich zu einer herkömmlichen Olefin-Syntheseanlage ein größerer Teil der Energie und/oder der Nettoenergie, die von der Olefin-Syntheseanlage und/oder einem oder mehreren Abschnitten davon benötigt wird, durch eine nicht kohlenstoffbasierte und/oder erneuerbare Energiequelle und/oder Elektrizität bereitgestellt wird.

Die Erfindung stellt sich die Aufgabe, entsprechende Verfahren und Anlagen zu verbessern und dabei insbesondere auch an das jeweilige Energieangebot am Standort der Anlage anpassbar auszugestalten.

### Offenbarung der Erfindung

Diese Aufgabe wird durch ein Verfahren und eine Anlage zur Herstellung von Ethylen und/oder anderen Olefinen durch Steamcracken mit den Merkmalen der jeweiligen unabhängigen Patentansprüche gelöst. Ausgestaltungen sind jeweils Gegenstand der abhängigen Patentansprüche und der nachfolgenden Beschreibung.

Insgesamt schlägt die vorliegende Erfindung ein Verfahren zur Herstellung von Ethylen und/oder anderen Olefinen durch Steamcracken vor, bei dem ein oder mehrere Cracker, also Spaltöfen, die in der üblichen Weise mit Konvektions- und Strahlungszone ausgebildet sein können, mit einem paraffinhaltigen Feed wie Naphtha oder Ethan bzw. entsprechenden Gemischen oder beliebigen anderen, in der Fachwelt bekannten oder vorteilhaften Einsätzen beschickt werden und bei dem dem einen oder den mehreren Crackern ein Rohgas entnommen wird, wobei das Rohgas zumindest zum Teil einer Aufbereitung unterworfen wird, die eine Rohgasverdichtung und eine thermische Trennung unter Verwendung eines Ethan- und/oder Ethylenkältemittels (C2-Kältemittel) und eines Propan- und/oder Propylenkältemittels (C3-Kältemittel) umfasst. Die genannten Kältemittel können insbesondere zum Kondensieren von Gasgemischen, zum Aufkochen von Sumpfverdampfern von Trennkolonnen oder in entsprechenden Kopfkondensatoren eingesetzt werden.

Ist hier von einem "Ethan- und/oder Ethylenkältemittel" oder "C2-Kältemittel" bzw. einem "Propan- und/oder Propylenkältemittel" bzw. "C3-Kältemittel" die Rede, kann es sich um entsprechende Reinstoffe oder Gemische der genannten Komponenten handeln. Es können jeweils, typischerweise in geringeren Mengen von weniger als 10%, auch andere Komponenten enthalten sein.

Für die Rohgasverdichtung wird im Rahmen der vorliegenden Erfindung ein Rohgasverdichter verwendet, das C2-Kältemittel wird unter Verwendung eines C2-Kältemittelverdichters verdichtet, und das C3-Kältemittel wird unter Verwendung eines C3-Kältemittelverdichters verdichtet. Zu weiteren Details eines entsprechenden Verfahrens, das auch weiter unten nochmals exemplarisch erläutert wird, sei auf den eingangs zitierten Stand der Technik ausdrücklich verwiesen. Wie erwähnt, werden der C2-Kältemittelverdichter und der C3-Kältemittelverdichter vereinfacht auch als C2-Kältemittelverdichter (ERC) bzw. C3-Kältemittelverdichter (PRC) bezeichnet.

Erfindungsgemäß weist der Rohgasverdichter zwei serielle Verdichterstränge auf, und jeder dieser Verdichterstränge, der C2-Kältemittelverdichter und der C3-Kältemittelverdichter werden jeweils zumindest zu einem Teil unter Verwendung elektrischer Antriebe betrieben. Zur Vermeidung von Missverständnissen sei darauf hingewiesen, dass unter der Angabe, dass bestimmte Verdichter bzw. Verdichterstränge "jeweils" unter Verwendung elektrischer Antriebe angetrieben werden, verstanden werden soll, dass einer der Verdichterstränge mit einem ersten der elektrischen Antriebe, der andere Verdichterstrang mit einem zweiten der elektrischen Antriebe, der C2-Kältemittelverdichter mit einem dritten der elektrischen Antriebe und der C3-Kältemittelverdichter mit einem vierten der elektrischen Antriebe angetrieben wird. Im Rahmen der vorliegenden Erfindung umfasst einer der Verdichterstränge des Rohgasverdichters, insbesondere der stromaufwärtige Verdichterstrang, insbesondere zwei Verdichterstufen auf, der andere Verdichterstrang insbesondere drei. Allgemeiner gesprochen weisen die Verdichterstränge insbesondere eine unterschiedliche Anzahl an Verdichterstufen auf.

Die erfindungsgemäß vorgeschlagene Betriebsweise bietet, wie erfindungsgemäß erkannt wurde, besondere Vorteile gegenüber aus dem Stand der Technik bekannten Antrieben, beispielsweise mittels Kondensations-Dampfturbinen nach API 612 oder anderen. Bei einer derartigen herkömmlichen Betriebsweise, deren nachfolgende Beschreibung die Erfindung in keiner Weise einschränken soll, wird beispielsweise unter Verwendung von Abwärme der Cracker Höchstdruckdampf (HHP-Dampf), d.h. Dampf auf einem Druckniveau von 90 bis 130 bar und einem Temperaturniveau von 450 bis 540 °C, bereitgestellt und zum Antrieb des Rohgasverdichters verwendet. Der Höchstdruckdampf wird dabei typischerweise über einen Hochdruckteil der Antriebsturbine des Rohgasverdichters entspannt. Ein signifikanter Anteil des Dampfes wird anschließend als Hochdruckdampf (HP-Dampf), d.h. Dampf auf einem Druckniveau von 35 bis 50 bar und einem Temperaturniveau von 250 bis 400 °C, extrahiert und den Antriebsturbinen des C2-Kältemittelverdichters und des C3-Kältemittelverdichters zugeführt. Je nach Bedarf weiterer Verbraucher werden zudem auch Mitteldruckdampf (MP-Dampf) bzw. Niederdruckdampf (LP-Dampf), also Dampf auf einem Druckniveau von 15 bis 25 bar und einem Temperaturniveau von 200 bis 250 °C bzw. Dampf auf einem Druckniveau von 3 bis 8 bar und einem Temperaturniveau von 150 bis 190 °C, erzeugt. Zur Hebung der Gesamtenergienbilanz kann zusätzlicher Dampf aus Hochdruckdampferzeugern oder von extern importiert werden. Ebenso kann hierbei ein Export von Dampf über die Anlagengrenze zur Bilanzierung vorgesehen sein.

Die energetische Bilanzierung des soeben beschriebenen herkömmlichen Verfahrens muss unter Beachtung der Restriktionen erfolgen, welche sich aus dem Leistungsbedarf der drei großen Verbraucher, d.h. des Rohgasverdichters, des C2-Kältemittelverdichters und des C3-Kältemittelverdichters, ergeben. Dies erfordert i.d.R. die Installation einer Hochdruckdampferzeugung umfassend einen entsprechenden Dampfkessel, um die Kopplung von Höchst- und Hochdruckdampfbedarf bilanztechnisch zu lösen. Dies erhöht den baulichen Aufwand. Zudem bleibt die Kondensationswärme des Dampfes weitgehend ungenutzt, vielmehr wird der Abdampf der Turbinen typischerweise mittels Kühlwasser mit hohem apparativem Aufwand kondensiert. Höchst- und Hochdruckdampferzeugung sind mit erheblichen Kohlendioxidemissionen verbunden, eine Einbindung alternativer (ggfs. kohlendioxidneutraler) Energie für den Antrieb der Verdichter ist herkömmlicherweise nicht vorgesehen oder möglich.

Durch den Einsatz der vorliegenden Erfindung ergibt sich durch den durch die Verwendung der elektrischen Antriebe sehr viel größeren Unabhängigkeit beim Betrieb der Verdichter eine Flexibilisierung der Ethylenerzeugung bzw. der Erzeugung anderer Olefine hinsichtlich der Dampf- und Kohlendioxidbilanzierung. Insbesondere steht die starre Kopplung der Höchstdruckdampfnutzung in der Rohgasverdichtung und der anschließenden Hochdruckdampfnutzung in der Verdichtung der Kältemittel im Rahmen der vorliegenden Erfindung der Flexibilisierung nicht mehr im Wege.

Gemäß einer besonders bevorzugten Ausgestaltung der vorliegenden Erfindung wird unter Verwendung von Abwärme des oder der Cracker weiterhin Höchstdruckdampf bereitgestellt. Dieser wird jedoch vorteilhafterweise zumindest zu einem Teil aus der Anlage exportiert und/oder zumindest teilweise ohne Verwendung zum Antrieb des Rohgasverdichters, des C2-Kältemittelverdichters und des C3-Kältemittelverdichters als Wärmequelle für andere Verfahrensschritte verwendet.

Der Höchstdruckdampf kann in einer besonders bevorzugten Ausgestaltung der vorliegenden Erfindung oder alternativ zu den erfindungsgemäß vorgeschlagenen Maßnahmen in einer Adaptionseinheit (sogenannte Let-Down-Station) hinsichtlich Druck und/oder Temperatur für den erwähnten Export und/oder den Verwendungszweck angepasst werden. In der Adaptionseinheit kann auch insbesondere anfallende Kondensationswärme für weitere Zwecke, beispielsweise zur Einspeisung in ein Fernwärmenetz, genutzt werden.

Das erfindungsgemäß eingesetzte Verfahren kann gemäß einer besonders bevorzugten Ausgestaltung ohne Verwendung eines Hochdruckdampfkessels durchgeführt werden. Mit anderen Worten kann durch den Einsatz der vorliegenden Erfindung ein entsprechender Hochdruckdampfkessel in Entfall kommen, so dass sich der Bedarf an fossilen Energieträgern verringert und damit die Kohlendioxidbilanz verbessert. Grundsätzlich kann im Rahmen der vorliegenden Erfindung an beliebigen Stellen eine Einbindung von kohlendioxidneutraler Energie erfolgen, wodurch sich eine erhebliche Flexibilisierung hinsichtlich des Kohlendioxid-Footprints ergibt.

Der Rohgasverdichter weist, wie erwähnt, erfindungsgemäß zwei serielle Verdichterstränge auf, also individuell antreibbare bauliche Einheiten, wobei jeder der Verdichterstränge des Rohgasverdichters sowie der C2-Kältemittelverdichter und der C3-Kältemittelverdichter jeweils mittels zumindest teilweise identische Leistungsmerkmale aufweisender elektrischer Antriebe betrieben werden. Vorteilhafterweise können die Antriebe auch im Wesentlichen baulich identisch ausgebildet sein. Auf diese Weise erhöht sich die Anzahl an Gleichteilen und die Erstellung einer entsprechenden Anlage wird im Sinne eines Standardisierungskonzepts deutlich verbessert.

Die zumindest teilweise identische Leistungsmerkmale aufweisenden elektrischen Antriebe werden dabei als baugleiche drehzahlvariable Antriebe bereitgestellt und diese werden jeweils über Frequenzumrichter gespeist. Jeder der seriellen Verdichterstränge der Rohgasverdichtung, der C2-Kältemittelverdichter und der Kältemittelverdichter sind in ihrem Drehmoment aneinander angeglichen. Auf diese Weise lässt sich eine entsprechende Standardisierung bei gleichzeitiger Flexibilität in der Drehzahl erreichen. Hierbei können insbesondere vier Frequenzumrichter bereitgestellt sein, von denen zu jedem Zeitpunkt des Anlagenbetriebs zwei oder auch alle vier zur Speisung der elektrischen Antriebe eingesetzt werden können. Ein fünfter Frequenzumrichter kann aus Redundanzgründen bereitgehalten werden, insbesondere für notwendige Wartungs- und/oder Reparaturarbeiten.

Durch den Einsatz der vorliegenden Erfindung kann für die Antriebe insbesondere auf baugleiche Maschinen zurückgegriffen werden, so dass sich insbesondere die Lagerhaltung der für diese Maschinen erforderlichen (Gleich-)Teile und die Wartung vereinfachen. Die vorliegende Erfindung beruht auf der Erkenntnis, dass die Verwendung baugleicher Antriebe trotz der damit verbundenen Schwierigkeiten und entsprechender Bedenken in der Fachwelt dennoch möglich und vorteilhaft ist. Mit der Erfindung wird damit insbesondere eine Verbesserung in der Erstellung und im Betrieb entsprechender Anlagen erzielt, wobei die erfindungsgemäß bzw. gemäß Ausgestaltungen der vorliegenden Erfindung vorgeschlagenen Maßnahmen ausgehend vom Stand der Technik nicht naheliegend waren.

Die Angleichung der Leistung der Antriebe ist nicht trivial, da Rohgasverdichter, Ethylen- und Propylenverdichter erheblich unterschiedliche Leistungen aufweisen. Ferner ist das Verhältnis der Leistung von Ethylenverdichter und Propylenverdichter variabel, und zwar abhängig von Feed (Einsatz) und Prozessführung (Abtrennung von schwereren Kohlenwasserstoffen, insbesondere mit fünf und mehr Kohlenstoffatomen. Der Rohgasverdichter weist typischerweise fünf Stufen auf, was prima facie gegen eine Teilbarkeit spricht. Die Druckverhältnisse am Rohgasverdichter können nur bedingt verschoben werden, da hohe Druckverhältnisse der einzelnen Stufen zu hohen Temperaturen und damit zum Risiko von Fouling führen könnten. Dies hätte den Fachmann davon abgehalten, ohne Kenntnis der Ausgestaltungen der Erfindung eine entsprechende Lösung in Betracht zu ziehen.

Im Rahmen von Ausgestaltungen der vorliegenden Erfindung kann insbesondere eine Aufteilung des Rohgasverdichters in zwei und drei Stufen in der oben erläuterten Weise und eine Verschiebung der Druckverhältnisse im Rahmen der gegebenen Beschränkungen vorgenommen werden, darüber hinaus kann insbesondere auch eine Verwendung von Fremdkälte erfolgen, wie sie gemäß einer Ausgestaltung der Erfindung vorgesehen ist. Diese führt ihrerseits auch zu einer erwünschten Belastung eines dabei insbesondere verwendeten Propylenverdichters und daher zur Angleichung von Propylen- und Ethylenverdichter.

Auch die Verwendung baugleicher drehzahlvariabler Antriebe ist ohne Kenntnis der Erfindung bzw. entsprechender Ausgestaltungen der Erfindung nicht naheliegend, weil nicht allein die Leistung, sondern vielmehr auch das Drehmoment angeglichen werden muss. Das bedeutet, dass die Verdichter entweder ähnlich schnell drehen, oder Getriebe eingesetzt werden müssen (Wandlung von Drehzahl und Drehmoment). Ohne Angleichung des Drehmoments lassen sich die Motoren nicht standardisieren, selbst bei gleicher Leistung. Daher kommt vorliegend insbesondere ein oder mehrere Getriebe zum Einsatz.

Vorteile von Ausgestaltungen der der Erfindung ergeben sich insbesondere durch die Bereitstellung einer Redundanz n + 1 mit der Verwendung eines zusätzlichen Frequenzumrichters. Diese lässt sich in anderen Ausgestaltungen der Erfindung auch mit unterschiedlichen Frequenzumrichtern und/oder Antrieben erzielen, allerdings unter Inkaufnahme von entsprechenden Anpassungen bei Wirkungsgrad, Ersatzteilhaltung und Bemessung. Der zusätzliche Frequenzumrichter muss hier dem größten der vier regulär eingesetzten Frequenzumrichter entsprechen und ohne Lastangleichung würde dieser zwangsläufig größer als die mittlere Leistung bei vier gleichen Antrieben.

Die vier Frequenzumrichter können dabei paarweise mit vollständiger Redundanz (2 x 100%) installiert werden wobei die beiden Frequenzumrichter jedes Paars im Teillastbetrieb (2 x 50%) parallel oder im Umschaltbetrieb (abwechselnd) mit Vollast (jeweils 1 x 100%) betrieben werden können. Im Fall von zwei unabhängigen Versorgungsnetzen können die Frequenzumrichter vorteilhaft auf die beiden Netze verschaltet werden. Dieses Redundanzkonzept bietet sich insbesondere an, wenn Spannungseinbrüche/Ausfälle in den Versorgungsnetzen erwartet werden und Umschaltzeiten zur Erhöhung der Anlagenverfügbarkeit minimiert werden sollen.

Für leistungsstarke Antriebe jenseits der Leistungsgrenze eines Frequenzumrichters ist zudem ein 3 x 50% Redundanzkonzept denkbar, bei dem zwei parallel betriebene Frequenzumrichter die Summenleistung von 100% bereitstellen.

In einer Ausführungsform des erfindungsgemäßen Verfahrens sind die vier Frequenzumrichter und der fünfte Frequenzumrichter jeweils als Teil einer Frequenzumrichteranordnung bereitgestellt, wobei der fünfte Frequenzumrichter mit seiner Frequenzumrichteranordnung während der Durchführung des Verfahrens in betriebsfähigem Zustand, insbesondere in einem unten erläuterten Hot Standby, d.h. in zumindest teilweise bestromtem Zustand, gehalten wird.

Gemäß einer Ausgestaltung umfasst die Frequenzumrichteranordnungen jeweils einen Eingangstransformator, insbesondere einen VSD-Transformator sowie einen Ein- und Ausgang, wobei der Ein- und Ausgang (der Eingang insbesondere über den Eingangstransformator) mittels Schalteinheiten mit einer Stromquelle und dem jeweiligen Antrieb verbunden sind. Durch ein selektives Ansteuern insbesondere der Schalteinheiten am Ausgang kann der jeweilige Frequenzumrichter selektiv anstelle eines anderen mit dem Antrieb verbunden werden.

Drehzahlvariable elektrische Antriebe nach heutigem Standard haben eine mittlere Betriebsdauer bis zum Ausfall bzw. zwischen Reparaturen (engl. Mean time between Failures, MTBF bzw. to Repair, MTTR) von etwa 10 Jahren. Da aber für den Anlagenbetrieb alle drei Verdichter (CGC, ERC und PRC) zugleich benötigt werden, reduziert sich die Verfügbarkeit (beispielsweise bestimmt mittels eines Zuverlässigkeitsblockdiagramms), sodass ein branchenüblicher ununterbrochener Anlagenbetrieb von 5 Jahren schwer zu realisieren ist. Die Standardisierung der Antriebe erlaubt es jedoch, zum Teil ohnehin notwendige Hauptersatzteile betriebsfähig im "Cold-Standby" oder "Hot-Standby" zu installieren. Daher kann bei einem Ausfall vorteilhafterweise ohne (nennenswerte) Verzögerung zwischen entsprechenden Einheiten umgeschaltet werden. Hierdurch kann die MTBF bzw. MTTR im Rahmen der vorliegenden Erfindung deutlich, beispielsweise auf bis zu 20 Jahre erhöht werden.

In der erläuterten Ausgestaltung der Erfindung wird also ein fünfter Frequenzumrichter vorteilhafterweise inklusive Peripheriegeräten (Kühlung, Transformator, Schalteinrichtungen) betriebsfertig installiert und derart verschaltet, dass er als Redundanz für jeden der vier bestehenden Frequenzumrichter genutzt werden kann. Für die Umschaltung kann eine temporäre Abschaltung der Maschine bzw. Stillstand in Betracht gezogen werden ("Cold Standby"). Alternativ kann im laufenden Betrieb umgeschaltet werden, wobei die Unterbrechung des Antriebsmomentes zu minimieren ist. Umschaltzeiten von weniger als 500 ms sind technisch möglich und anzustreben, optional ist die Umschaltsequenz regelungstechnisch mit der Pumpschutzregelung der Verdichter zu koppeln (Feed Forward-Signal zum Anti-Surge Regler).

Entsprechende Transformatoren und Frequenzumrichter sind in Ausgestaltung der Erfindung insbesondere sogenannte "Captial Spare"-Ersatzteile, d.h. diese werden bereits bei der Installation einer entsprechenden Anlage bereitgestellt und während des Betriebs fortwährend bereitgehalten. Sie zählen damit zu den Investitionskosten. Nimmt man diese Ersatzteile, wie in Ausgestaltungen der Erfindung vorgesehen, nicht ins Lager, sondern stellt diese als "installierte" Ersatzteile bereit, sind diese in sehr kurzer Zeit, beispielsweise innerhalb der erwähnten 500 ms einsatzbereit.

Ein besonderer Vorteil der vorliegenden Erfindung besteht auch darin, dass der Rohgasverdichter, der C2-Kältemittelverdichter und der C3-Kältemittelverdichter ebenerdig aufgestellt werden können, da keine Tischfundamente erfordernden Kondensatoren notwendig sind.

Vorteilhafterweise kann in dem Rohgasverdichter eine Zwischenkühlung zumindest zeitweise unter Verwendung von Fremdkälte vorgenommen werden, z.B. durch den Einsatz von Propan- oder Propylenkälte (C3-Kälte). Prinzipiell kann diese Zwischenkühlung vor jeder Stufe erfolgen. Eine Vorkühlung insbesondere der vierten Stufe ist besonders günstig, da hierdurch gleiche Leistung der ersten bis dritten Stufe einerseits und der vierten und fünften Stufe andererseits erreicht werden kann, unter Vermeidung unzulässig hoher Austrittstemperaturen. In diesem Zusammenhang kann eine abweichende Aufteilung von Verdichterstufen auf Antriebe vorgesehen sein, als sie oben in einem Beispiel erläutert wurde. Dies ist insbesondere im Sinne der beschriebenen Standardisierung besonders günstig. Die Fremdkälte für die Zwischenkühlung kann im Rahmen der vorliegenden Erfindung unter Verwendung zumindest eines Teils des C2-Kältemittels und/oder des C3-Kältemittels bereitgestellt werden, indem entsprechendes Kältemittel aus den jeweiligen Kältemittelkreisläufen ausgekoppelt wird.

In Ausgestaltungen der vorliegenden Erfindung kann die erwähnte Zwischenkühlung entweder permanent oder aber immer (nur) dann unter Verwendung von Fremdkälte vorgenommen werden, wenn eine Kühlwassertemperatur und/oder Wassereinspritzung als nicht ausreichend und/oder nicht funktionsfähig erkannt wird. Hieraus ergibt sich die Möglichkeit, Betriebskosten zu optimieren.

Im Rahmen der vorliegenden Erfindung ist in einer besonders bevorzugten Ausgestaltung vorgesehen, die Antriebsleistungen der elektrischen Antriebe des Rohgasverdichters, des C2-Kältemittelverdichter und des C3-Kältemittelverdichters durch eine Verschiebung von Kälteleistung zwischen einem C2-Kältemmittelkreislauf, in dem das C2-Kältemittel eingesetzt wird, und eines C3-Kältemittelkreislaufs, in dem das C3-Kältemittel eingesetzt wird, und/oder durch die erläuterte Einbindung von C3-Kälte als zusätzliche Zwischenkühlung des Rohgasverdichters einzustellen, um den Leistungsbedarf möglichst zu vergleichmäßigen.

Die Erfindung erstreckt sich auch auf eine Anlage zur Herstellung von Ethylen und/oder anderen Olefinen durch Steamcracken, mit einem oder mehreren Crackern, die zur Beschickung mit einem paraffinhaltigen Feed und zur Bereitstellung eines Rohgases eingerichtet sind, wobei die Anlage dafür eingerichtet ist, das Rohgas zumindest zum Teil einer Aufbereitung zu unterwerfen, die eine Rohgasverdichtung und eine thermische Trennung unter Verwendung eines C2-Kältemittels und eines C3-Kältemittels umfasst, wobei für die Rohgasverdichtung ein Rohgasverdichter bereitgestellt ist, zur Verdichtung des C2-Kältemittels ein C2-Kältemittelverdichter bereitgestellt ist, und zur Verdichtung des C3-Kältemittels ein C3-Kältemittelverdichter bereitgestellt ist. Zu den erfindungsgemäßen Merkmalen wird auf den entsprechenden unabhängigen Patentanspruch verwiesen.

Zu Merkmalen und Vorteilen einer entsprechenden Anlage und vorteilhafter Ausgestaltungen hiervon sei auf die obigen Erläuterungen bezüglich des erfindungsgemäß vorgeschlagenen Verfahrens und seiner Ausgestaltungen ausdrücklich verwiesen, da diese in entsprechender Weise auch für die Anlage und deren Ausgestaltungen gelten. Dies ist insbesondere auch bei einer Anlage der Fall, die zur Durchführung eines Verfahrens eingerichtet ist, wie es oben in unterschiedlichen Ausgestaltungen erläutert wurde.

Die Erfindung wird nachfolgend unter Bezugnahme auf die beigefügte Zeichnung erläutert, welche eine Ausgestaltung der Erfindung veranschaulicht.

### Figurenbeschreibung

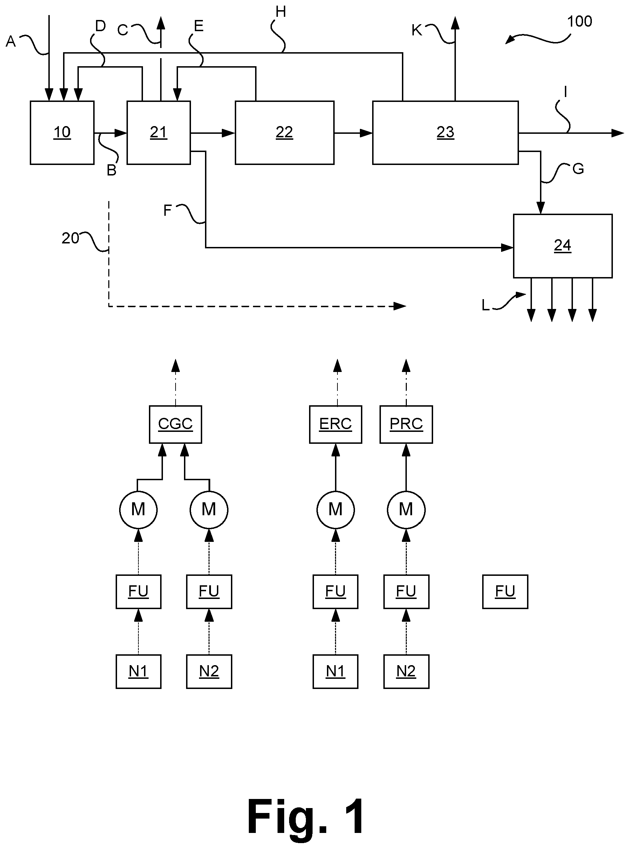

In Figur 1 ist ein Verfahren gemäß einer Ausgestaltung der vorliegenden Erfindung in Form eines vereinfachten Prozessflussdiagramms veranschaulicht und insgesamt mit 100 bezeichnet. Wenngleich die nachfolgenden Erläuterungen sich auf ein Verfahren und entsprechende Verfahrensschritte beziehen, gelten diese für eine entsprechende Anlage und deren Komponenten in gleicher Weise.

In dem Verfahren 100 werden ein oder mehrere Cracker (Spaltöfen) 10 eingesetzt, die mit einem Feed A (sowie Dampf, wie hier nicht gesondert veranschaulicht) beschickt werden, und dem oder denen ein Rohgas B entnommen wird.

Das Rohgas B wird zumindest zum Teil einer hier insgesamt mit 20 bezeichneten Aufbereitung unterworfen, die in an sich bekannter Weise einen Quenchschritt 21 unter Abscheidung von Pyrolyseöl C sowie eine Verdichtung (Rohgasverdichtung) 22 und eine thermische Trennung 23 unter Verwendung eines C2-Kältemittels und eines C3-Kältemittels umfasst. In dem Quenchschritt 21 kann Dampf D bereitgestellt und in den oder die Cracker 10 zurückgeführt werden. In der Verdichtung 22 kann Pyrolysebenzin E abgetrennt und beispielsweise in den Quenchschritt 21 zurückgeführt werden. In dem Quenchschritt 21 können ferner Kohlenwasserstoffe F mit drei und mehr Kohlenstoffatomen abgetrennt und in einen entsprechenden Verarbeitungsschritt 24 überführt werden, in dem auch entsprechende Kohlenwasserstoffe G aus der thermischen Trennung 23 eingespeist werden können. In der thermischen Trennung 23 kann ein Ethanstrom H bereitgestellt und in den oder die Spaltöfen 10 zurückgeführt werden. Ein Ethylenstrom I kann als Produkt ausgeführt werden. Sogenanntes Tailgas K, das insbesondere Methan und Wasserstoff enthält, wird ausgeschleust. Weitere Produktströme, insgesamt mit L bezeichnet, werden in dem weiteren Verarbeitungsschritt 24 bereitgestellt.

Wie hier symbolisch in einem unteren Bereich der Figur 1 veranschaulicht, wird für die Rohgasverdichtung 22 ein mit CGC bezeichneter Verdichter verwendet, das Ethylenkältemittel wird unter Verwendung eines mit ERC bezeichneten C2-Kältemittelverdichters verdichtet und das Propylenkältemittel wird unter Verwendung eines mit PRC bezeichnetenC3-Kältemittelverdichters verdichtet. Der Rohgasverdichter CGC, der C2-Kältemittelverdichter ERC und der C3-Kältemittelverdichter PRC werden jeweils zumindest zu einem Teil unter Verwendung eines oder mehrerer elektrischer Antriebe M betrieben.

Genauer umfasst in der hier veranschaulichten Ausgestaltung der Erfindung der Rohgasverdichter CGC zwei serielle Verdichterstränge (nicht gesondert dargestellt), wobei jeder der Verdichterstränge des Rohgasverdichters CGC sowie der C2-Kältemittelverdichter ERC und der C3-Kältemittelverdichter PRC jeweils mittels zumindest teilweise identische Leistungsmerkmale aufweisender elektrischer Antriebe betrieben werden. Diese können hier als baugleiche drehzahlvariable Antriebe bereitgestellt werden und jeweils über einen Frequenzumrichter FU gespeist werden. Insgesamt sind dabei in dem hier veranschaulichten Beispiel fünf Frequenzumrichter FU bereitgestellt, von denen zu jedem Zeitpunkt zwei (im paarweisen Volllast-Wechselbetrieb) oder vier (im paarweisen Teillast-Betrieb) zur Speisung der elektrischen Antriebe M eingesetzt werden und einer aus Redundanzgründen bereitgehalten wird. Im hier veranschaulichten Beispiel werden die Frequenzumrichter FU in der dargestellten Weise an unterschiedliche Netze bzw. Netzteile N1, N2 angebunden. Die Erfindung ist durch das hier veranschaulichte Beispiel nicht beschränkt.

Der Dampf D oder anderer in dem Verfahren bereitgestellter Dampf kann insbesondere Höchstdruckdampf umfassen, der exportiert und/oder zumindest teilweise ohne Verwendung zum Antrieb des Rohgasverdichters RGC, des C2-Kältemittelverdichters ERC und des C3-Kältemittelverdichters PRC als Wärmequelle für andere Verfahrensschritte verwendet wird. Der Höchstdruckdampf kann einer hier allgemein mit 50 angegebenen Adaptionseinheit hinsichtlich Druck und/oder Temperatur für den Export und/oder den Verwendungszweck angepasst werden. In der der Adaptionseinheit 50 anfallende Kondensationswärme kann in der erläuterten Weise genutzt werden.

Zu weiteren Ausgestaltungen der Erfindung, die alle auch in dem in Figur 1 dargestellten Verfahren 100 einsetzbar sind, sei auf die obigen Erläuterungen nochmals ausdrücklich verwiesen.

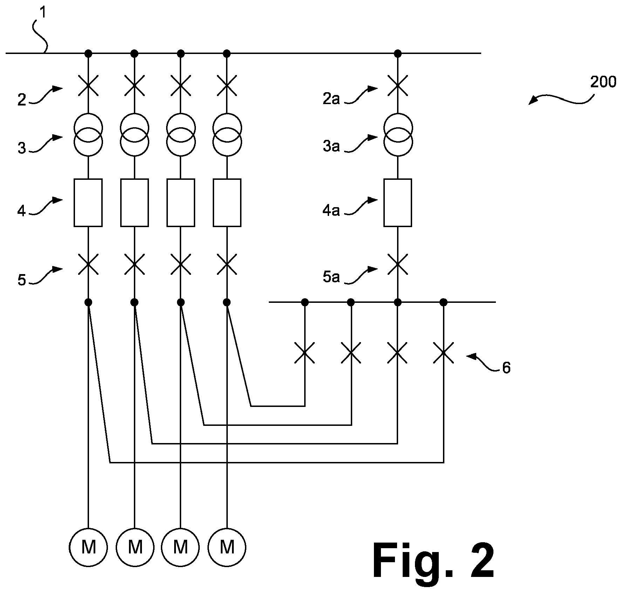

In Figur 2 ist eine Anordnung 200 gemäß einer Ausgestaltung der Erfindung veranschaulicht. Es ist jeweils eine Vielzahl vorzugsweise identisch ausgebildeter Elemente veranschaulicht, die daher nur jeweils einmal mit entsprechenden Bezugszeichen versehen sind.

An einer Stromschiene 1 sind über Schalteinrichtungen 2 jeweils Eingangstransformatoren (insbesondere VSD-Transformatoren) angebunden, die jeweils mit Frequenzumrichtern, die hier mit 4 bezeichnet und insbesondere als indirekte Umrichter mit Gleichspannung im Zwischenkreis (VSI) ausgebildet sind, verbunden sind. Wiederum über Schalteinrichtungen 5 sind die jeweiligen Antriebe angebunden. Die im linken Teil der Figur dargestellte Gruppe aus Schalteinrichtungen 2a und 5a und Eingangstransformator 3a und Frequenzumrichter 4a kann mittels einer der Schalteinrichtungen 6 wahlweise auf einen der Antriebe M aufgeschaltet werden, wenn der Antrieb M über die entsprechende Schalteinrichtung 5 von dem ursprünglich verbundenen Frequenzumrichter getrennt wurde. Die Schalteinrichtung 2a kann dabei im gesamten Betrieb geschlossen bleiben, so dass der Eingangstransformator 3a und Frequenzumrichter 4a im "Hot Standby" gehalten werden können.

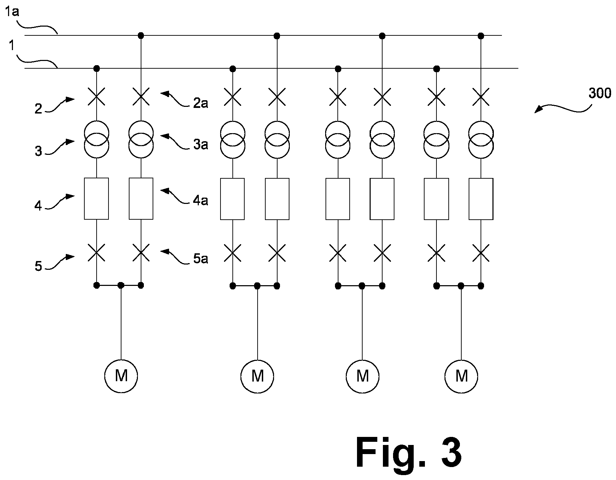

In Figur 3 ist eine Anordnung 300 gemäß einer weiteren Ausgestaltung der Erfindung veranschaulicht. Auch hier ist jeweils eine Vielzahl vorzugsweise identisch ausgebildeter Elemente veranschaulicht, die daher nur jeweils einmal mit entsprechenden Bezugszeichen versehen sind.

Im Unterschied zu der in Figur 2 veranschaulichten Anordnung 200 ist in der in Figur 3 veranschaulichten Anordnung 300 ein abweichendes Redundanzkonzept veranschaulicht, bei dem jeder der Antriebe M alternativ mit einem von zwei Strängen aus Schalteinrichtungen 2 und 5, Eingangstransformator 3 und Frequenzumrichter 4 (bzw. 2a, 5a, 3a und 4a, nur am linken Antrieb M entsprechend bezeichnet) angebunden werden kann.

## Patentansprüche

1. Verfahren (100) zur Herstellung von Ethylen und/oder anderen Olefinen durch Steamcracken, bei dem ein oder mehrere Cracker (10) mit einem paraffinhaltigen Feed beschickt werden und dem einen oder mehreren Crackern (10) ein Rohgas entnommen wird, wobei das Rohgas zumindest zum Teil einer Aufbereitung (20) unterworfen wird, die eine Rohgasverdichtung (22) sowie eine thermische Trennung (23) unter Verwendung eines C2-Kältemittels und eines C3-Kältemittels umfasst, wobei für die Rohgasverdichtung (22) ein Rohgasverdichter (CGC) verwendet wird, wobei das C2-Kältemittel unter Verwendung eines C2-Kältemittelverdichters (ERC) verdichtet wird, und wobei das C3-Kältemittel unter Verwendung eines C3-Kältemittelverdichters (PRC) verdichtet wird, **dadurch gekennzeichnet, dass** der Rohgasverdichter (CGC) zwei serielle Verdichterstränge umfasst, und dass jeder dieser Verdichterstränge, der C2-Kältemittelverdichter (ERC) und der C3-Kältemittelverdichter (PRC) jeweils zumindest zu einem Teil unter Verwendung elektrischer Antriebe (M) betrieben werden, die zumindest teilweise identische Leistungsmerkmale aufweisen, jeweils über Frequenzumrichter (FU) gespeist werden, und als baugleiche drehzahlvariable Antriebe bereitgestellt sind, wobei jeder der seriellen Verdichterstränge der Rohgasverdichtung (22), der C2-Kältemittelverdichter (ERC) und der C3-Kältemittelverdichter (PRC) in ihrem Drehmoment aneinander angeglichen sind.

2. Verfahren nach Anspruch 1, bei dem unter Verwendung von Abwärme Höchstdruckdampf bereitgestellt und exportiert und/oder zumindest teilweise ohne Verwendung zum Antrieb des Rohgasverdichters (RGC), des C2-Kältemittelverdichters (ERC) und des C3-Kältemittelverdichters (PRC) als Wärmequelle für andere Verfahrensschritte verwendet wird.

3. Verfahren nach Anspruch 2, bei dem der Höchstdruckdampf in einer Adaptionseinheit (50) hinsichtlich Druck und/oder Temperatur für den Export und/oder den Verwendungszweck angepasst wird.

4. Verfahren nach Anspruch 3, wobei in der Adaptionseinheit (50) anfallende Kondensationswärme genutzt wird.

5. Verfahren nach einem der vorstehenden Ansprüche, das ohne Verwendung eines Hochdruckdampfkessels betrieben wird.

6. Verfahren nach einem der vorstehenden Ansprüche, bei dem vier Frequenzumrichter (FU) bereitgestellt sind, von denen zu jedem Zeitpunkt zwei oder vier zur Speisung der elektrischen Antriebe eingesetzt werden, und bei dem insbesondere ein fünfter Frequenzumrichter (FU) aus Redundanzgründen bereitgehalten wird.

7. Verfahren nach Anspruch 6, bei dem die vier Frequenzumrichter (FU) und der fünfte Frequenzumrichter (FU) jeweils als Teil einer Frequenzumrichteranordnung bereitgestellt sind, wobei der fünfte Frequenzumrichter (FU) mit seiner Frequenzumrichteranordnung während der Durchführung des Verfahrens in betriebsfähigem Zustand gehalten wird.

8. Verfahren nach Anspruch 7, bei dem die Frequenzumrichteranordnungen jeweils einen Eingangstransformator sowie einen Ein- und Ausgang umfasst, wobei der Ein- und Ausgang über Schalteinheiten mit einer Stromquelle und dem jeweiligen Antrieb verbunden sind.

9. Verfahren nach einem der vorstehenden Ansprüche, bei dem der Rohgasverdichter (CGC), der C2-Kältemittelverdichter (ERC) und der C3-Kältemittelverdichter (PRC) ebenerdig aufgestellt sind.

10. Verfahren nach einem der vorstehenden Ansprüche, bei dem in dem Rohgasverdichter (CGC) eine Zwischenkühlung zumindest zeitweise unter Verwendung von Fremdkälte vorgenommen wird.

11. Verfahren nach Anspruch 10, bei dem die Fremdkälte für die Zwischenkühlung unter Verwendung zumindest eines Teils des C2-Kältemittels und/oder des C3-Kältemittels bereitgestellt wird.

12. Verfahren nach einem der Ansprüche 10 oder 11, bei dem die Zwischenkühlung dann unter Verwendung von Fremdkälte vorgenommen wird, wenn eine Kühlwassertemperatur und/oder Wassereinspritzung als nicht ausreichend und/oder nicht funktionsfähig erkannt wird.

13. Verfahren nach einem der vorstehenden Ansprüche, bei dem Antriebsleistungen der elektrischen Antriebe des Rohgasverdichters (CGC), des C2-Kältemittelverdichter (ERC) und des C3-Kältemittelverdichters (PRC) durch eine Verschiebung von Kälteleistung zwischen einem Ethylenkältemittelkreislauf, in dem das Ethylenkältemittel eingesetzt wird, und eines Propylenkältemittelkreislaufs, in dem das Propylenkältemittel eingesetzt wird, und/oder Einbindung von Propylenkälte als zusätzliche Zwischenkühlung des Rohgasverdichters (CGC) eingestellt werden.

14. Anlage zur Herstellung von Ethylen und/oder anderen Olefinen durch Steamcracken, mit einem oder mehreren Cracker (10), die zur Beschickung mit einem paraffinhaltigen Feed und zur Bereitstellung eines Rohgases eingerichtet sind, wobei die Anlage dafür eingerichtet ist, das Rohgas zumindest zum Teil einer Aufbereitung (20) zu unterwerfen, die eine Verdichtung (22) und eine thermische Trennung (23) unter Verwendung eines C2-Kältemittels und eines C3-Kältemittels umfasst, wobei für die Rohgasverdichtung (22) ein Rohgasverdichter (CGC) bereitgestellt ist, zur Verdichtung des C2-Kältemittels ein C2-Kältemittelverdichter (ERC) bereitgestellt ist, und zur Verdichtung des C3-Kältemittels ein C3-Kältemittelverdichter (PRC) bereitgestellt ist, **dadurch gekennzeichnet, dass** der Rohgasverdichter (CGC) zwei serielle Verdichterstränge umfasst, und dass zur Bereitstellung jeweils zumindest eines Teils der Antriebsleistung der zwei Verdichterstränge des Rohgasverdichters (CGC), des C2-Kältemittelverdichters (ERC) und des C3-Kältemittelverdichter (PRC) jeweils elektrische Antriebe (M) bereitgestellt sind, die zumindest teilweise identische Leistungsmerkmale aufweisen, jeweils über Frequenzumrichter (FU) speisbar sind, und als baugleiche drehzahlvariable Antriebe bereitgestellt sind, wobei jeder der seriellen Verdichterstränge der Rohgasverdichtung (22), der C2-Kältemittelverdichter (ERC) und der C3-Kältemittelverdichter (PRC) in ihrem Drehmoment angeglichen sind.

## Claims

1. Method (100) for producing ethylene and/or other olefins by steam cracking, in which one or more crackers (10) are loaded with a paraffin-containing feed and a crude gas is extracted from the one or more crackers (10), wherein the crude gas is subjected at least in part to a treatment (20) comprising a crude gas compression (22) and a thermal separation (23) using a C2 refrigerant and a C3 refrigerant, wherein a crude gas compressor (CGC) is used for the crude gas compression (22), wherein the C2 refrigerant is compressed using a C2 refrigerant compressor (ERC), and wherein the C3 refrigerant is compressed using a C3 refrigerant compressor (PRC), **characterized in that** the crude gas compressor (CGC) comprises two serial compressor trains, **and in that** each of these compressor trains, the C2 refrigerant compressor (ERC) and the C3 refrigerant compressor (PRC) are each operated at least in part using electric drives (M), which have at least partially identical performance features, are each supplied with power via frequency converters (FU), and are provided as structurally identical variable-speed drives, wherein each of the serial compressor trains of the crude gas compression (22), the C2 refrigerant compressor (ERC) and the C3 refrigerant compressor (PRC) are matched to one another in terms of their torque.

2. Method according to claim 1, wherein, using waste heat, ultrahigh-pressure steam is provided and exported and/or used at least in part as a heat source for other method steps, without being used to drive the crude gas compressor (RGC), the C2 refrigerant compressor (ERC) and the C3 refrigerant compressor (PRC).

3. Method according to claim 2, wherein the ultrahigh-pressure steam is adapted in an adaptation unit (50) with respect to pressure and/or temperature for the export and/or the intended use.

4. Method according to claim 3, wherein condensation heat obtained in the adaptation unit (50) is used.

5. Method according to any of the preceding claims, which is conducted without using a high-pressure steam boiler.

6. Method according to any of the preceding claims, wherein four frequency converters (FU) are provided, of which two or four are used at any time to supply the electric drives with power, and wherein in particular a fifth frequency converter (FU) is kept ready for redundancy reasons.

7. Method according to claim 6, wherein the four frequency converters (FU) and the fifth frequency converter (FU) are each provided as part of a frequency converter arrangement, wherein the fifth frequency converter (FU) with its frequency converter arrangement is held in operational state during the execution of the method.

8. Method according to claim 7, wherein the frequency converter arrangements each comprise an input transformer and an input and output, wherein the input and output are connected to a current source and the relevant drive via switching units.

9. Method according to any of the preceding claims, wherein the crude gas compressor (CGC), the C2 refrigerant compressor (ERC), and the C3 refrigerant compressor (PRC) are set up at ground level.

10. Method according to any of the preceding claims, wherein intermediate cooling is carried out in the crude gas compressor (CGC) at least temporarily using external refrigeration.

11. Method according to claim 10, wherein the external refrigeration is provided for the intermediate cooling using at least a portion of the C2 refrigerant and/or the C3 refrigerant.

12. Method according to claim 10 or 11, wherein the intermediate cooling is carried out using external refrigeration if a cooling water temperature and/or water injection is recognized as being insufficient and/or not functional.

13. Method according to any of the preceding claims, wherein the drive powers of the electric drives of the crude gas compressor (CGC), the C2 refrigerant compressor (ERC) and the C3 refrigerant compressor (PRC) are adjusted by a displacement of cooling power between an ethylene refrigerant circuit, in which the ethylene refrigerant is used, and a propylene refrigerant circuit, in which the propylene refrigerant is used, and/or the integration of propylene refrigeration as additional intermediate cooling of the crude gas compressor (CGC).

14. Plant for producing ethylene and/or other olefins by steam cracking, having one or more crackers (10), which are designed for being loaded with a paraffin-containing feed and for providing a crude gas, wherein the plant is designed to subject the crude gas at least in part to a treatment (20) comprising a compression (22) and a thermal separation (23) using a C2 refrigerant and a C3 refrigerant, wherein a crude gas compressor (CGC) is provided for the crude gas compression (22), a C2 refrigerant compressor (ERC) is provided for the C2 refrigerant compression, and a C3 refrigerant compressor (PRC) is provided for the C3 refrigerant compression, **characterized in that** the crude gas compressor (CGC) comprises two serial compressor trains, **and in that,** to provide in each case at least a portion of the drive power of the two compressor trains of the crude gas compressor (CGC), of the C2 refrigerant compressor (ERC) and of the C3 refrigerant compressor (PRC), electric drives (M) are provided in each case, which have at least partially identical performance features, are each supplied with power via frequency converters (FU), and are provided as structurally identical variable-speed drives, wherein each of the serial compressor trains of the crude gas compression (22), the C2 refrigerant compressor (ERC) and the C3 refrigerant compressor (PRC) are matched to one another in terms of their torque.

## Revendications

1. Procédé (100) de production d'éthylène et/ou d'autres oléfines par vapocraquage, dans lequel un ou plusieurs craqueurs (10) sont chargés avec un flux paraffinique et un gaz brut est extrait du craqueur ou des craqueurs (10), le gaz brut étant au moins en partie soumis à un traitement (20) qui comprend une compression de gaz brut (22) ainsi qu'une séparation thermique (23) à l'aide d'un réfrigérant C2 et d'un réfrigérant C3, un compresseur de gaz brut (CGC) étant utilisé pour la compression de gaz brut (22), le réfrigérant C2 étant comprimé à l'aide d'un compresseur de réfrigérant C2 (ERC), et le réfrigérant C3 étant comprimé à l'aide d'un compresseur de réfrigérant C3 (PRC),
**caractérisé en ce que** le compresseur de gaz brut (CGC) comprend deux trains de compresseurs en série, **et en ce que** chacun de ces trains de compresseurs, le compresseur de réfrigérant C2 (ERC) et le compresseur de réfrigérant C3 (PRC) fonctionnent respectivement au moins en partie à l'aide d'entraînements électriques (M) qui présentent des caractéristiques de performance au moins partiellement identiques, sont respectivement alimentés par l'intermédiaire de convertisseurs de fréquence (FU), et sont fournis en tant qu'entraînements à vitesse de rotation variable de construction identique, chacun des trains de compresseurs en série de la compression de gaz brut (22), le compresseur de réfrigérant C2 (ERC) et le compresseur de réfrigérant C3 (PRC) étant équilibrés en termes de couple.

2. Procédé selon la revendication 1, dans lequel de la vapeur à pression maximale est fournie à l'aide d'une chaleur résiduelle puis exportée et/ou est utilisée, comme source de chaleur pour d'autres étapes de procédé, au moins partiellement sans aide, pour l'entraînement du compresseur de gaz brut (RGC), du compresseur de réfrigérant C2 (ERC) et du compresseur de réfrigérant C3 (PRC).

3. Procédé selon la revendication 2, dans lequel la vapeur à pression maximale est adaptée dans une unité d'adaptation (50) en ce qui concerne la pression et/ou la température, pour être exportée et/ou utilisée.

4. Procédé selon la revendication 3, dans lequel une chaleur de condensation générée est utilisée dans l'unité d'adaptation (50).

5. Procédé selon l'une des revendications précédentes, lequel est mis en oeuvre sans l'aide d'une chaudière de haute pression.

6. Procédé selon l'une des revendications précédentes, dans lequel quatre convertisseurs de fréquence (FU) sont fournis, dont deux ou quatre sont utilisés à tout moment pour l'alimentation des entraînements électriques, et dans lequel en particulier un cinquième convertisseur de fréquence (FU) est tenu à disposition à des fins de redondance.

7. Procédé selon la revendication 6, dans lequel les quatre convertisseurs de fréquence (FU) et le cinquième convertisseur de fréquence (FU) sont respectivement fournis comme faisant partie d'un agencement de convertisseur de fréquence, le cinquième convertisseur de fréquence (FU) et son agencement de convertisseur de fréquence étant maintenus en état de marche pendant la réalisation du procédé.

8. Procédé selon la revendication 7, dans lequel les agencements de convertisseur de fréquence comprennent respectivement un transformateur d'entrée ainsi qu'une entrée et une sortie, l'entrée et la sortie étant reliées à une source d'énergie et à l'entraînement respectif par l'intermédiaire d'unités de commutation.

9. Procédé selon l'une des revendications précédentes, dans lequel le compresseur de gaz brut (CGC), le compresseur de réfrigérant C2 (ERC) et le compresseur de réfrigérant C3 (PRC) sont installés au niveau du sol.

10. Procédé selon l'une des revendications précédentes, dans lequel un refroidissement intermédiaire est effectué au moins par moments dans le compresseur de gaz brut (CGC) à l'aide d'un froid extérieur.

11. Procédé selon la revendication 10, dans lequel le froid extérieur destiné au refroidissement intermédiaire est fourni à l'aide d'au moins une partie du réfrigérant C2 et/ou du réfrigérant C3.

12. Procédé selon l'une des revendications 10 ou 11, dans lequel le refroidissement intermédiaire est ensuite effectué à l'aide d'un froid extérieur lorsqu'une température d'eau de refroidissement et/ou une injection d'eau est identifiée comme insuffisante et/ou comme inefficace.

13. Procédé selon l'une des revendications précédentes, dans lequel des performances de propulsion des entraînements électriques du compresseur de gaz brut (CGC), du compresseur de réfrigérant C2 (ERC) et du compresseur de réfrigérant C3 (PRC) sont réglées par un décalage de la performance de réfrigération entre un cycle réfrigérant à l'éthylène, dans lequel le réfrigérant à l'éthylène est utilisé, et un cycle réfrigérant au propylène, dans lequel le réfrigérant au propylène est utilisé, et/ou par l'intégration d'une réfrigération au propylène comme refroidissement intermédiaire supplémentaire du compresseur de gaz brut (CGC).

14. Installation de production d'éthylène et/ou d'autres oléfines par vapocraquage, comportant un ou plusieurs craqueurs (10) qui sont conçus pour être chargés avec un flux paraffinique et pour fournir un gaz brut, l'installation étant conçue pour soumettre le gaz brut au moins en partie à un traitement (20) qui comprend une compression (22) et une séparation thermique (23) à l'aide d'un réfrigérant C2 et d'un réfrigérant C3, un compresseur de gaz brut (CGC) étant fourni pour la compression de gaz brut (22), un compresseur de réfrigérant C2 (ERC) étant fourni pour la compression du réfrigérant C2, et un compresseur de réfrigérant C3 (PRC) étant fourni pour la compression du réfrigérant C3, **caractérisée en ce que** le compresseur de gaz brut (CGC) comprend deux trains de compresseurs en série, **et en ce que** des entraînements électriques (M) respectifs sont fournis pour la fourniture respectivement d'au moins une partie de la performance de propulsion des deux trains de compresseurs du compresseur de gaz brut (CGC), du compresseur de réfrigérant C2 (ERC) et du compresseur de réfrigérant C3 (PRC), lesquels entraînements électriques présentent des caractéristiques de fonctionnement au moins partiellement identiques, peuvent être alimentés respectivement par l'intermédiaire de convertisseurs de fréquence (FU) et sont fournis en tant qu'entraînements à vitesse de rotation variable de construction identique, chacun des trains de compresseurs en série de la compression de gaz brut (22), le compresseur de réfrigérant C2 (ERC) et le compresseur de réfrigérant C3 (PRC) étant équilibrés en termes de couple.
